(19) Europäisches Patentamt
European Patent Office
Office européen des brevets

(11) Publication number: 0 068 268 B1

# (12) EUROPEAN PATENT SPECIFICATION

(45) Date of publication of patent specification: 02.05.85

(51) Int. Cl.[4]: A 61 K 31/70 // C07H19/16, C07H19/20

(21) Application number: 82105194.3

(22) Date of filing: 14.06.82

## (54) Enhancer of anti-tumour effect.

(30) Priority: 18.06.81 JP 94676/81

(43) Date of publication of application: 05.01.83 Bulletin 83/01

(45) Publication of the grant of the patent: 02.05.85 Bulletin 85/18

(84) Designated Contracting States: BE CH DE FR GB LI NL SE

(56) References cited:
CHEMICAL ABSTRACTS, vol. 94, no. 21, 25th May 1981, page 767, no. 175450q, Columbus, Ohio, US

(73) Proprietor: Yamasa Shoyu Kabushiki Kaisha
10-1, Araoi-Cho 2-Chome
Choshi-Shi Chiba-Ken (JP)

(72) Inventor: Nakatsugawa, Shigekazu
38-159, Ooshima, Momoyama-Cho
Fushimi-Ku Kyoto-Shi, Kyoto-Fu (JP)

(74) Representative: Dr. Elisabeth Jung Dr. Jürgen Schirdewahn Dr. Gerhard Schmitt-Nilson Dr. Gerhard B. Hagen Dipl.-Ing. Peter Hirsch
P.O. Box 40 14 68
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 068 268 B1

**Description**

Background of the invention

Field of the art

This invention relates to an enhancer of anti-tumor effect.

In the art of treatment of tumors, there have been many developments from various aspects. In radiotherapy, as a branch of these developments, there have also been attempts to improve the results of therapy. As one method, it is proposed to improve geometrical dose distribution by use of such methods as radiation of accelerated heavy ion particals or π meson. Another approach now under the development is to enhance selectively the sensitivity of tumor cells under hypoxic conditions, which are most resistant to radiotherapy among tumors, by utilizing a hypoxic cell sensitizer. Alternatively, combination treatments incorporating a method utilizing other anti-tumor factors, such as hyperthermia or chemotherapy have been attempted.

However, in the method for improvement of geometrical dose distribution, it is necessary to use enormous funds for installation of an accelerator and auxiliary equipments necessary for practicing the method as well as a large personnel including expert engineers and physicians. Other methods also involve drawbacks such as great damage to normal cells. For example, misonidazole, which is a hypoxic cell sensitizer, has neurotoxicity, and hence it is difficult to administer it in a large quantity, whereby no great radiosensitizing effect can be expected at concentrations available in clinical use, its effect being small in a low dose range (less than 1,000 rad) as employed in a routine therapy.

On the other hand, in the field of chemotherapy of tumors, multiple anti-tumor agents have been combined to be used for the following purposes and effects:

1) By using in combination a number of different agents selected from those of alkylating agents, antimetabolites, antibiotics and alkaroids, which show mutually no cross resistancy and are different in mechanism of action, the anti-tumor effect can be enhanced additively or synergistically against tumors which are composed of a mixture of tumor cells different in sensitivity to various agents.

2) By using in combination anti-tumor agents different in the way they attack tumor cells which are proliferating at random, various stages in the cell cycle of tumor cells can be widely attacked to ensure complete killing of tumor cells.

3) By using not only agents different in mechanism of action but also those having relatively similar mechanisms of action, a synergistic effect can be expected. For example, by using in combination a number of agents which are blocking a series of steps participating in DNA synthesis, a strong synergistic effect can be exhibited.

4) Each anti-tumor agent has its specific side effect. Thus, by using in combination a number of agents with different side effects each in a dosage less than the limit above which side effects appear, the anti-tumor effect can be expected to be increased additively or synergistically while the side effects are dispersed.

By such a multi-agent combination treatment, it has been made possible to obtain an effect which could not be produced by using a single anti-tumor agent. However, each of the agents used in combination in such an application is an anti-tumor agent which can be independently used.

There have also been various attempts to use in combination with an anti-tumor agent a compound which does not *per se* have an anti-tumor effect for the purpose of strengthening the effect of the anti-tumor agent by preventing the anti-tumor agent from being inactivated in bodies. For example, it is known to use cytidine or uridine in combination with 1-β-D-arabinofuranosylcytosine (hereinafter referred to as "araC"), as disclosed in Japanese Patent Laid-Open Publication No. 24150/1980. It is also known to use tetrahydrouridine, which is an inhibitor against cytidinedeaminase, in combination with araC, as disclosed in Cancer Research Vol. 30, p. 2166—2172, 1970. Further, there is known another method wherein 5-fluorouracil (hereinafter referred to as "5-FU") or a derivative thereof is combined with a pyrimidine compound such as, for example, uracil, cytosine, thymine, orotic acid, 5-bromouracil, 5-iodouracil, 1-acetyluracil, 1-(2-tetrahydrofuryl)-uracil, 3-benzoyluracil, 1-cyclohexycarbamoyluracil, 1-n-hexycarbamoyluracil, uridine, 2'-deoxyuridine, 5-bromo-2'-deoxyuridine, cytidine, or 2'-deoxycytidine.

On the other hand, in the field of radiotherapy, the tumor cells with radioresistance under hypoxic conditions are at a quiescent stage, and also there has been observed a phenomenon wherein potentially lethal damage repair (hereinafter referred to as "PLDR") is markedly manifested particularly in the cells at a quiescent stage. By inhibiting PLDR of such tumor cells, it is possible to increase the therapeutical effect of radiotherapy. We have investigated the possibility of radio-sensitization by inhibiting PLDR with the use of a chemical. During the course of this investigation, 3'-deoxyadenosine (cordycepin, herein referred to as "3'-dAdo") was confirmed to have a PLDR-inhibiting ability. However, 3'-dAdo is readily inactivated by adenosinedeaminase in bodies. We found that the PLDR-inhibiting ability of 3'-dAdo can be strengthened and prolonged by using in combination therewith 2'-deoxycoformycin, which is an inhibitor against adenosinedeaminase. However, there is also a report that 2'-deoxycoformycin may cause damage to the immune systems, and 3'-dAdo is also known to have various side effects. Thus, it would be very desirable to have a radiosensitizing substance which is more stable, lower in toxicity, and more effective than 3'-dAdo.

The PLDR phenomenon of tumor cells is observed not only in the field of radiotherapy but also in the

treatment with a chemotherapeutics such as Bleomycin or 5-FU, as reported in the Journal of the National Cancer Institute, Vol. 50, No. 2, p. 529—533, 1973. Accordingly, a pharmaceutical agent capable of inhibiting repair from damage of tumor cells can also enhance the anti-tumor effect not only of radiation but also of chemotherapeutics.

Summary of the invention

In view of the above described state of the art, we have made extensive studies with the aim of obtaining a radiosensitizing agent having a PLDR-inhibiting activity with low toxicity and good stability. As a result, it has now been found that a $N^6$-acyl derivative of 3′-dAdo has a PLDR-inhibiting activity and excellent radiosensitizing activity. It has also been found that these $N^6$-acyl-3′-dAdo derivatives can also exhibit excellent effect in strengthening anti-tumor effect in treatment of malignant tumors with chemotherapeutics. The present invention has been accomplished on the basis of such findings. Thus, the present invention provides a pharmaceutical agent to be used for strengthening the anti-tumor effect in treatment of malignant tumors by irradiation and/or an anti-tumor agent.

Chemical Abstracts Vol. 94, No. 21, 25th May, 1981, page 767, No. 175450 g discloses that $N^6$-acylcordycepin derivatives have adenosine deaminase inhibitory activity, although the original Jpn. Kokai Tokkyo Koho 80, 149,299 abstracted in the C.A. does not show that $N^6$-acylcordycepin derivatives have inhibitory activity against adenosine deaminase while it shows that they are resistant to adenosine deaminase. It is not disclosed by the C.A. or the original Japanese Kokai that these cordycepin derivatives have an activity for enhancing anti-tumor action of radiation and chemotherapeutic agents.

While the mechanism in which the pharmaceutical agent of the present invention acts on tumors has not yet been completely clarified, the present pharmaceutical agent may be considered to be a pharmaceutical which will enhance the anti-tumor effect of radiation or a chemotherapeutic through acting (e.g. inhibitory action) on changes in nucleic acid metabolism such as PLDR (e.g. repair of damage in DNA) caused by treatment with radiation or chemotherapeutics.

In one aspect of the present invention, the present invention relates to a preparation of an enhancer of anti-tumor effect which comprises an $N^6$-acyl-3′-deoxyadenosine derivative of a formula (I):

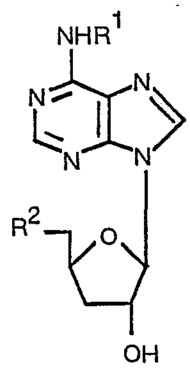

(I)

wherein $R^1$ represents an acyl group containing 22 to 26 carbon atoms and $R^2$ represents a hydroxyl group, a phosphoric acid residue or a phosphoric acid salt residue, and a pharmaceutically acceptable carrier. In another aspect of the present invention, the present invention relates to a chemotherapeutic composition for treating tumors which comprises an anti-tumor agent, an enhancer of anti-tumor effect which is an $N^6$-acyl-3′-deoxyadenosine derivative of the formula (I) and a pharmaceutically acceptable carrier.

In still another aspect of the present invention, the present invention relates to an $N^6$-acyl-3′-deoxyadenosine derivative of the formula (I) for use as an enhancer of anti-tumor effect in a tumor-bearing animal under the anti-tumor treatment.

The term "animal" as herein used means a human being or a lower animal.

The wording "under the anti-tumor treatment" means the state wherein a tumor-bearing animal is being subjected to physical, chemical or physicochemical treatment for suppressing tumors or the state wherein there is retained in that animal an influence due to such a treatment.

Accordingly and more specifically, an $N^6$-acyl-3′-deoxyadenosine compound of the formula (I) is used as an enhancer in an anti-tumor treatment which comprises irradiation to a tumor site of the animal, the enhancer being used before, simultaneously with, or after that irradiation. According to another embodiment an $N^6$-acyl-3′-deoxyadenosine derivative of the formula (I) is used as an enhancer in an anti-tumor treatment which comprises administration of an anti-tumor agent the enhancer being used before, simultaneously with or after the administration of the anti-tumor agent.

Detailed description of the invention

Enhancers

The enhancers in accordance with the present invention are $N^6$-acyl-3′-deoxyadenosine (dAdo) derivatives of a specified group. The enhancers may alternatively be regarded as repair-inhibiting agents, and both terms are herein used interchangeably.

The $N^6$-acyl-3′-dAdo derivatives are represented by the formula:

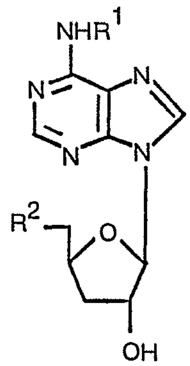

(I)

wherein $R^1$ represents an acyl group which preferably contains 2 to 26 carbon atoms, more preferably 2 to 22 carbon atoms and $R^2$ hydroxyl group, phosphoric acid residue or a phosphoric acid salt residue.

More specifically, typical examples of the compounds may include $N^6$-acetyl-3′-dAdo, $N^6$-propionyl-3′-dAdo, $N^6$-butyryl-3′-dAdo, $N^6$-hexanoyl-3′-dAdo, $N^6$-heptanoyl-3′-dAdo, $N^6$-octanoyl-3′-dAdo, $N^6$-nonanoyl-3′-dAdo, $N^6$-decanoyl-3′-dAdo, $N^6$-lauroyl-3′-dAdo, $N^6$-palmitoyl-3′-dAdo, $N^6$-stearoyl-3′-dAdo, and 5′-phosphoric acid ester derivatives thereof. When $R^2$ is a phosphoric acid salt, the kind of the salt may be any of those which are pharmaceutically acceptable, including, for example, alkali metal salts such as those of lithium, sodium, potassium, alkaline earth metal salts such as those of calcium, magnesium, and ammonium salts.

An $N^6$-acyl-3′-dAdo derivative can be prepared according to the method in which 3′-dAdo or its 5′-phosphoric acid ester is allowed to react in a pyridine type solvent with an acid anhydride or an acid halogenide corresponding to the acyl group in a desired compound to synthesize an $N^6$, $O^{2'}$, $O^{5'}$-triacyl-3′-dAdo or an $N^6$, $O^{2'}$-diacyl-3′-dAdo-5′-phosphate, which reaction is followed by hydrolysis of the intermediate to eliminate the acyl groups at the sugar moieties.

Enhancement of anti-tumor effect

The pharmaceutical agent according to the present invention, when it is a preparation of an enhancer of anti-tumor effect which is free of an anti-tumor agent, may be used for the purpose of enhancing the anti-tumor effect in the treatment of a malignant tumor, for which radiotherapy or chemotherapy by anti-tumor agents is to be applied, in combination with the treatments by these therapeutical methods.

In the case where the pharmaceutical agent of the present invention is used as a radiosensitizing agent for the purpose of enhancing the effect of radiotherapy, it may be administered before or after exposure, or even during exposure, if the occasion permits it, to the irradiation in radiotherapy. As to radiotherapy *per se*, the use of specific method and conditions is not required, but conventional radiotherapy techniques may be employed. By the use of the enhancer of the present invention in combination, it has become possible to apply radiotherapy with irradiation in the region of lower dosage than in the prior art. As the ionizing radiations for radiotherapy, those generally employed such as X-rays, lineac high energy X-rays, betatron 32 MeV electron beams or $^{60}Co$-γ-rays may be used.

When used for the purpose of enhancing the anti-tumor effect in chemotherapy by an anti-tumor agent, the enhancer of the present invention may be administered simultaneously with, after, or before administration of the anti-tumor agent. Anti-tumor agents, of which anti-tumor effects are to be enhanced by the pharmaceutical agent of the present invention, are exemplified typically by substances having activity similar to irradiations, including also those which can induce changes in nucleic acid metabolism such as repair phenomenon like PLDR in tumor cells on which an $N^6$-acyl-3′-dAdo can act after treatment therewith. Examples of anti-metabolites are methotrexate; 6-mercaptopurine; 5-FU and its derivatives, such as, for example, 5-fluorouridine, 5-fluoro-2′-deoxyuridine, 1-β-D-arabinofuranosyl-5-fluorocytosine, 1-(2-tetrahydrofuryl)-5-fluorouracil (hereinafter referred to as "FT-207"), 1-(n-hexylcarbamoyl)-5-fluorouracil, 1-ethoxymethyl-5-fluorouracil, 1-ethoxycarbonyl-5-fluorouracil, and 5-fluoro-5′-deoxyuridine; and araC and its derivatives, such as, for example, cyclocytidine, $N^4$-palmitoyl araC, $N^4$-stearoyl araC, $N^4$-behenoyl araC, araC-5′-phospho-stearyl ester, and araC-5′-phospho-oleyl ester may be mentioned. Examples of anti-tumor antibiotics are Bleomycin; Neocarzinostatin; and Anthracycline type antibiotics, such as, for example, Daunomycin, Adriamycin, and Aclacinomycin. Examples of alkylating agents include nitrosourea, such as, for example, ACNU, BCNU, CCNU, MCCNU; 3′-[3-(2-chloroethyl)-3-nitrosoureido]-3′-deoxythymidine; and 3′-(3-methyl-3-nitrosoureido)-3′-deoxythymidine. These anti-tumor agents may be administered by any method and in any dosage which are not specifically limited in combination with the enhancer of the present invention, but optimum conditions may suitably be selected for each agent used.

The pharmaceutical agent according to another aspect of the present invention, when it is a chemotherapeutic composition, comprises an anti-tumor agent and the enhancer of anti-tumor effect in

combination. Anti-tumor agents and the enhancers of anti-tumor effect to be used are set forth hereinabove.

The pharmaceutical agents according to the present invention, irrespective of whether they are enhancers of anti-tumor agent or chemotherapeutic compositions, may comprise a pharmaceutically acceptable carrier. Examples of such carriers include lactose, magnesium stearate, talc, corn starch, "Witepsol" (Tradename, supplied by Dynamit Nobel Co., Germany), crystalline cellulose, distilled water and alcohols.

The method for administration of the pharmaceutical agent of the present invention may in general be either systemic administration or local administration. Various dosage unit forms can be selected depending on the purposes of therapy and the methods of administration. For example, as the form for systemic administration, an oral administration form such as tablet, capsule, granule or solution, or a non-oral administration form such as injection or suppository, can be used. On the other hand, as a local administration form, a slow-releasing-capsule, an ointment or an injection can be used. In the preparing of such a dosage unit form, it is possible to make a preparation according to a conventional method using a pharmaceutically acceptable carrier. Various modifications in preparation suitable for the object of the present invention may also be utilized.

The $N^6$-acyl-3'-dAdo derivative of the present invention is used in an amount effective for enhancement of anti-tumor activity. More specifically, the dosage of the pharmaceutical agent of the present invention per day, which may slightly differ depending on the active ingredient employed, in general, is desirably 20 to 3,000 mg for an oral administration, 0.5 to 500 mg for an injection, and 20 to 2,000 mg for a suppository, as determined from basic experiments on anti-tumor effectiveness. The optimum effective amount should be determined by judgement of a physician according to the irradiation used, its dosage, the anti-tumor agent used, its dosage, the conditions of disease or the affected part.

The pharmacological effects of the pharmaceutical agents of the present invention are shown below with data from the tests of radiosensitizing effect thereof.

Experiment 1

Radiosensitizing effect on the tumor cells in culture

Cells under stationary phase, prepared by planting $2\times10^5$ cells/well of Chinese hamster-HA-1 cells in a multiwell dish and exchanging cultural medium every day starting on the third day and thereafter were irradiated with 1,000 R of X-rays. Immediately after radiation, $N^6$-butyryl-3'-dAdo in Hanks' balanced salt solution were administered. Then, after elapse of various times, the cells were taken off with trypsin, and a suitable number of cells were transferred into petri dishes for testing colony forming ability and plating efficiencies. The percentages of survival were calculated with corrections by the plating efficiencies with the pharmaceutical agents alone. The results are shown in Table 1.

TABLE 1
Radiosensitizing effect of the enhancers in Chinese hamster-HA-1 cells

| Enhancer | Treatment conc. µg/ml | Plating efficiency (%) | | | |
|---|---|---|---|---|---|
| | | Immediately after irradiation | After 1 hour | After 4 hours | After 7 hours |
| $N^6$-butyryl -3'-dAdo | 0 | 0.94 | 2.7 | 3.6 | 5.0 |
| | 40 | | 1.4 | 2.2 | 2.4 |

As is apparent from Table 1, $N^6$-butyryl-3'-dAdo is found to inhibit repair (PLDR) after irradiation with X-rays. Substantially no toxicity was observed, as examined by plating efficiencies.

Experiment 2

Radiosensitizing effect on experimental tumor in mice

EMT-6 tumor cells ($2\times10^5$) were inoculated intradermally into the right thighs of BALB/c-strain female mice (8 weeks old, 6 or more mice for each group). When the tumor size reached 5.5 to 7.5 mm in diameter after inoculation of the tumor cells, local irradiation with a 32 MeV electron beam was carried out at 1,500 rad under no anesthesia and thereafter $N^6$-butyryl-3'-dAdo dissolved in physiological saline solution, was administered intraperitoneally to each mouse in 100 mg/kg. On the 9th day and 20th day after these treatments, t' tumor sizes were measured in tri-dimensional diameters, and compared with the control group with respect to the following items:

1) Mean tumor diameter $= \left( \dfrac{\text{Maximum longitudinal diameter} + \text{Maximum lateral diameter} + \text{Maximum height (diameter)}}{3} \right)$

2) Diameter ratio $= \dfrac{\text{Mean tumor diameter}}{\text{Mean tumor diameter at the time of irradiation}}$

The results are shown in Table 2, in which "cure" means that the tumors completely vanished during the observation period.

TABLE 2
Sensitizing effect of enhancer on X-ray therapy of EMT-6 tumors

| Treatment | Mean tumor diameter at the time of irradiation (mm) | 9 days after treatment | | | 20 days after treatment | | | Cured cases |
|---|---|---|---|---|---|---|---|---|
| | | Mean tumor diameter (mm) | Diameter ratio | Sensitizing effect* | Mean tumor diameter (mm) | Diameter ratio | Sensitizing effect* | |
| Irradiation (1500 rad) alone (n=8) | 6.61±0.57 | 6.40±0.93 | 0.97±0.07 | 1 | 7.83±2.01 | 1.11±0.24 | 1 | 4/8 |
| Irradiation+ $N^6$-butyryl-3′-dAdo (n=9) | 6.43±0.29 | 5.36±1.13 | 0.83±0.18 | 0.86 | 0.0±0.0 | — | 0.00 | 9/9 |

$$\text{* Sensitizing effect} = \frac{\text{Diameter ratio when the pharmaceutical agent of the invention is used}}{\text{Diameter ratio when the pharmaceutical agent of the invention is not used (irradiation alone)}}$$

Experiment 3

Radiosensitizing effect on experimental tumors in mice

EMT-6 tumor cells ($2\times10^5$) were inoculated intradermally into the right thighs of BALB/c-strain female mice (8 weeks old). When the tumor sizes reached 5.5 to 8.0 mm after inoculation of the tumor cells, irradiation was locally carried out with 1,000 rad to 2,700 rad of 32 MeV electron beam under no anesthesia, and thereafter 3'-dAdo and $N^6$-butyryl-3'-dAdo, each dissolved in physiological saline solution, were administered intraperitoneally in a dose of 100 mg/kg. After the treatments, observation was continued for longer than 90 days, and the cure percentages were determined for comparison with the control group.

1) $\text{Cure percent}=\frac{\text{Cure numbers on 90th day}}{\text{Number of mice at irradiation}}\times100$

2) $TCD_{50}$=50% Tumor control dose

The results are shown in Table 3.

TABLE 3
Sensitizing effect of enhancer on X-ray therapy of EMT-6 tumors

| Treatment | Cure number/Number treated (%) | | | | $TCD_{50}$ (ratio) |
|---|---|---|---|---|---|
| | 1,000 rad | 1,500 rad | 2,100 rad | 2,700 rad | |
| Control | 1/23 (4%) | 8/44 (18%) | 9/24 (38%) | 13/28 (47%) | 2400 (1.0) |
| 3'-dAdo | 3/18 (17) | 14/29 (48) | 11/14 (79) | 10/15 (67) | 1600 (0.67) |
| $N^6$-butyryl-3'-dAdo | 6/15 (40) | 13/20 (65) | 14/15 (93) | 16/16 (100) | 1080 (0.45) |

Experiment 4

Radiosensitizing effect on experimental tumors in mice

$RIF_1$ tumor cells ($2\times10^5$) were inoculated intradermally into the right thighs of C3H/Heston-strain female mice (8 weeks old). When the tumor sizes reached 6.0 to 10.0 mm after inoculation of the tumor cells, irradiation was carried out with 2,500 rad/min of an electron beam, and thereafter $N^6$-acetyl-3'-dAdo, $N^6$-butyrl-3'-dAdo, $N^6$-hexanoyl-3'-dAdo, and $N^6$-octanoyl-3'-dAdo, each dissolved in physiological saline solution, were administered intraperitoneally in a dose of 100 mg/kg. After the treatments, the tumor sizes in terms of diameters in three-axial directions were measured on the 10th day and the 20th day (9th day and the 21st day only in case of $N^6$-butyryl-3'-dAdo). The results are shown in Table 4.

TABLE 4
Sensitizing effect of enhancer on X-ray therapy of $RIF_1$ tumors

| Treatment | Mean tumor diameter at the time of irradiation (mm) | 10th day after treatment | | | 20th day after treatment | | |
|---|---|---|---|---|---|---|---|
| | | Mean tumor diameter (mm) | Diameter ratio | Sensitizing effect | Mean tumor diameter (mm) | Diameter ratio | Sensitizing effect |
| Irradiation (2500 rad) alone (n=7) | 6.83±0.52 | 8.07±0.79 | 1.18±0.06 | 1 | 12.00±0.65 | 1.78±0.07 | 1 |
| Irradiation+ $N^6$-acetyl-3′-dAdo (n=7) | 7.03±0.39 | 7.86±0.55 | 1.12±0.06 | 0.95 | 10.39±0.51 | 1.49±0.06 | 0.84 |
| Irradiation+ $N^6$-butyryl-3′-dAdo (n=7) | 7.44±0.19 | 6.71±0.63 | 0.90±0.08 | 0.76 | 10.28±0.36 | 1.35±0.08 | 0.76 |
| Irradiation+ $N^6$-hexanoyl-3′-dAdo (n=7) | 7.20±0.46 | 7.90±0.47 | 1.11±0.05 | 0.94 | 10.37±0.42 | 1.46±0.05 | 0.82 |
| Irradiation+ $N^6$-octanoyl-3′-dAdo (n=7) | 7.23±0.30 | 7.50±0.37 | 1.04±0.04 | 0.88 | 9.61±0.46 | 1.33±0.02 | 0.75 |

Experiment 5

Enhancement of the effect of anti-tumor agents on experimental tumors in mice

EMT-6 tumor cells ($1\times10^6$) were inoculated intradermally into the right thighs of BALB/c-strain mice (8 weeks old). On the 12th day after inoculation of the tumor cells and thereafter, each chemical in a physiological saline solution was administered intraperitoneally to each mouse four times a week in the indicated doses for respective agents. The pharmaceutical agents of the present invention were administered one hour after administration of the anti-tumor agents. After commencement of the treatment with pharmaceutical agents, the tumor sizes were measured every other day and compared with those of the control group.

The results are given in Table 5.

TABLE 5

Enhancing effect of enhancer on chemotherapy of EMT-6 tumors

| Treatment with anti-tumor agent | Treatment with pharmaceutical of the invention | Mean tumor diameter immediately before treatment (mm) | 14th day after treatment | | |
|---|---|---|---|---|---|
| | | | Mean tumor diameter (mm) | Diameter ratio (mm) | Enhancing effect |
| Control (n=6) | — | 6.00±0.89 | 9.20±1.78 | 1.57±0.40 (1.00) | |
| Control (n=5) | $N^6$-butyryl-3′-dAdo (50 mg/kg×7) | 7.60±0.56 | 9.92±2.45 | 1.36±0.32 (0.87) | |
| FT-207 100 mg/kg×7 (n=4) | — | 6.68±1.04 | 7.80±1.64 | 1.19±0.26 (0.76) | |
| FT-207 100 mg/kg×7 (n=5) | $N^6$-butyl-3′-dAdo (50 mg/kg×7) | 6.88±0.37 | 6.72±1.69 | 0.97±0.23 (0.62) | 0.94 |

$$\text{Enhancing effect} = \frac{\text{Diameter ratio when anti-tumor agent and pharmaceutical of the invention are used in combination}}{\text{(Diameter ratio when anti-tumor agent alone is used)} \times \text{(Diameter ratio when pharmaceutical of the invention alone is used)}}$$

Experiment 6

Acute toxicity test

Various quantities of $N^6$-butyryl-3′-dAdo were administered intraperitoneally to ICR mice (male, 8-weeks old, 10 mice for each group) in doses of 250 mg to 1,000 mg/kg, and the mice were subjected to observation for a week. As a result, the $LD_{50}$ of $N^6$-butyryl-3′-dAdo was found to be 710 mg/kg (640 mg/kg—788 mg/kg: 95% confidence limit). The $LD_{50}$ (i.p.) of 3′-dAdo was 280 mg/kg (241.4 mg/kg—324.8 mg/kg: 95% confidence limit).

Preparation 1

| | |
|---|---|
| $N^6$-butyryl-3′-dAdo | 100 mg |
| Lactose | 170 mg |
| Magnesium stearate | 3 mg |
| Crystalline cellulose | 57 mg |
| | 330 mg/capsule |

Capsules are prepared according to the above formulation.

Preparation 2

| | |
|---|---|
| $N^6$-butyryl-3′-dAdo | 100 mg |
| Lactose | 100 mg |
| Magnesium stearate | 2 mg |
| Talc | 3 mg |
| Hydroxypropylmethyl cellulose | 10 mg |
| | 215 mg/tablet |

Tablets are prepared according to the above formulation.

Preparation 3

| | |
|---|---|
| $N^6$-butyryl-3′-dAdo | 500 mg |
| Lactose | 240 mg |
| Corn starch | 250 mg |
| Hydroxypropylmethyl cellulose | 10 mg |
| | 1,000 mg/package |

Granules are prepared according to the above formulation.

Preparation 4

| | |
|---|---|
| $N^6$-butyryl-3′-dAdo | 100 mg |
| Tris-amino methane | 220 mg |
| Distilled water for injection | appropriate amount |
| | 10 ml/ampoule |

Injections are prepared according to the above formulation.

Preparation 5

| | |
|---|---|
| $N^6$-butyryl-3′-dAdo | 500 mg |
| Witepsol W-35 | 1,500 mg |
| | 2,000 mg/suppository |

Suppositories are prepared according to the above formulation.

Preparation 6

| | |
|---|---|
| $N^6$-butyryl-3′-dAdo | 100 mg |
| FT-207 | 100 mg |
| Lactose | 100 mg |
| Crystalline cellulose | 57 mg |
| Magnesium stearate | 3 mg |
| | 360 mg/capsule |

Capsules are prepared according to the above formulation.

Preparation 7

| | |
|---|---|
| $N^6$-butyryl-3′-dAdo | 100 mg |
| FT-207 | 100 mg |
| Lactose | 33 mg |
| Crystalline cellulose | 15 mg |
| Magnesium stearate | 2 mg |
| Talc | 3 mg |
| Corn starch | 14 mg |
| Hydroxypropylmethyl cellulose | 10 mg |
| | 277 mg/tablet |

Tablets are prepared according to the above formulation.

Preparation 8

| | |
|---|---|
| $N^6$-butyryl-3′-dAdo | 100 mg |
| FT-207 | 200 mg |
| Lactose | 580 mg |
| Corn starch | 90 mg |
| Hydroxypropylmethyl cellulose | 30 mg |
| | 1,000 mg/package |

Parvules are prepared according to the above formulation.

Preparation 9

| | |
|---|---|
| $N^6$-butyryl-3′-dAdo | 50 mg |
| FT-207 | 100 mg |
| Sodium carbonate | 440 mg |
| Sodium hydroxide | 35 mg |
| Distilled water for injection | appropriate amount |
| | 10 ml/ampoule |

Injections are prepared according to the above formulation.

Preparation 10

| | |
|---|---|
| $N^6$-butyryl-3′-dAdo | 300 mg |
| FT-207 | 750 mg |
| Witepsol® W-35 | 950 mg |
| | 2,000 mg/suppository |

Suppositories are prepared according to the above formulation.

**Claims**

1. A preparation of an enhancer of anti-tumor effect which comprises: an $N^6$-acyl-3′-deoxyadenosine derivative represented by the formula:

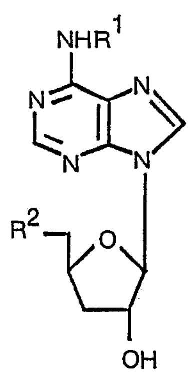

(I)

wherein $R^1$ represents an acyl group containing 2 to 26 carbon atoms and $R^2$ represents a hydroxyl group, a phosphoric acid residue or a phosphoric acid salt residue, and a pharmaceutically acceptable carrier.

2. The preparation as claimed in claim 1 in which the substituent $R^1$ contains 2 to 22 carbon atoms.

3. A chemotherapeutic composition for treating tumors which comprises an anti-tumor agent, an enhancer of anti-tumor effect which is an $N^6$-acyl-3′-deoxyadenosine derivative represented by the formula:

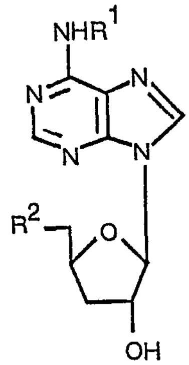

(I)

wherein $R^1$ represents an acyl group containing 2 to 26 carbon atoms and $R^2$ represents a hydroxyl group, a phosphoric acid residue or a phosphoric acid salt residue, and a pharmaceutically acceptable carrier.

4. The chemotherapeutic composition for treating tumors as claimed in claim 3 in which the anti-tumor agent is selected from anti-metabolites, anti-tumor antibiotics and alkylating agents.

5. An $N^6$-acyl-3'-deoxyadenosine derivative having the following formula:

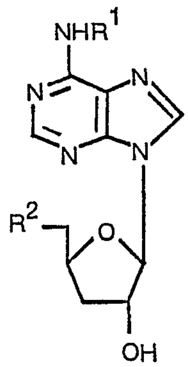

(I)

wherein $R^1$ represents an acyl group containing 2 to 26 carbon atoms and $R^2$ represents a hydroxyl group, a phosphoric acid residue or a phosphoric acid salt residue, for use as an enhancer of anti-tumor effect in a tumor-bearing animal under anti-tumor treatment.

6. A compound as claimed in claim 5 for use as an enhancer in an anti-tumor treatment which comprises irradiation of a tumor site of the animal, the enhancer being used before, simultaneously with, or after the irradiation.

7. A compound as claimed in claim 5 for use as an enhancer in an anti-tumor treatment before the animal is subjected to the irradiation.

8. A compound as claimed in claim 5 for use as an enhancer in an anti-tumor treatment after the animal has been subjected to the irradiation.

9. A compound as claimed in claim 5 for use as an enhancer in an anti-tumor treatment which comprises administration to the animal of an anti-tumor agent, the enhancer being used before, simultaneously with, or after the administration of the anti-tumor agent.

10. A compound as claimed in claim 5 for use as an enhancer in an anti-tumor treatment in which the anti-tumor agent is selected from anti-metabolites, anti-tumor antibiotics and alkylating agents.

**Revendications**

1. Préparation d'un renforçateur d'effet antitumoral qui consiste en:
un dérivé du type $N^6$-acyl-3'-désoxyadénosine représenté par la formule:

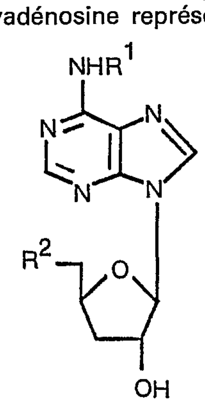

(I)

dans laquelle $R^1$ représente un groupe acyle comportant 2 à 26 atomes de carbone et $R^2$ représente un groupe hydroxyle, un résidu d'acide phosphorique ou un résidu de sel d'acide phosphorique, et un support pharmaceutiquement acceptable.

2. Préparation selon la revendication 1, dans laquelle le substituant $R^1$ comporte 2 à 22 atomes de carbone.

3. Composition chimiothérapique pour le traitement de tumeurs, qui comprend un agent antitumoral, un renforçateur d'effet antitumoral qui est un dérivé du type $N^6$-acyl-3'-désoxyadénosine représenté par la formule:

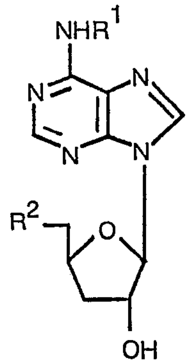

(I)

dans laquelle $R^1$ représente un groupe acyle comportant 2 à 26 atomes de carbone et $R^2$ représente un groupe hydroxyle, un résidu d'acide phosphorique ou un résidu de sel d'acide phosphorique, et un support pharmaceutiquement acceptable.

4. Composition chimiothérapique pour le traitement de tumeurs selon la revendication 3, dans laquelle l'agent antitumoral est choisi parmi les antimétabolites, les antibiotiques antitumoraux et les agents alkylants.

5. Dérivé du type $N^6$-acyl-3'-désoxyadénosine de formule suivante:

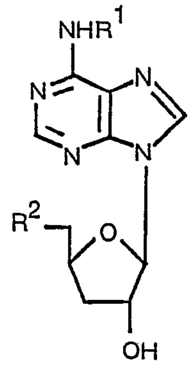

(I)

dans laquelle $R^1$ représente un groupe acyle comportant 2 à 26 atomes de carbone et $R^2$ représente un groupe hydroxyle, un résidu d'acide phosphorique ou un résidu de sel d'acide phosphorique, destiné à être utilisé en tant que renforçateur d'effet antitumoral chez un animal porteur de tumeur sous traitement antitumoral.

6. Composé selon la revendication 5 destiné à être utilisé en tant que renforçateur dans un traitement antitumoral qui comprend l'irradiation d'un site de tumeur de l'animal, le renforçateur étant utilisé avant, en même temps que, ou après l'irradiation.

7. Composé selon la revendication 5 destiné à être utilisé en tant que renforçateur dans un traitement antitumoral avant que l'animal ne soit soumis à l'irradiation.

8. Composé selon la revendication 5 destiné à être utilisé en tant que renforçateur dans un traitement antitumoral après que l'animal a été soumis à l'irradiation.

9. Composé selon la revendication 5 destiné à être utilisé en tant que renforçateur dans un traitement antitumoral qui comprend l'administration à l'animal d'un agent antitumoral, le renforçateur étant utilisé avant, en même temps que, ou après l'administration de l'agent antitumoral.

10. Composé selon la revendication 5 destiné à être utilisé en tant que renforçateur dans un traitement antitumoral dans lequel l'agent antitumoral est choisi parmi les antimétabolites, les antibiotiques antitumoraux et les agents alkylants.

**Patentansprüche**

1. Eine Zubereitung eines Verstärkungsmittels für die Antitumorwirkung, welches ein $N^6$-Acyl-3'-deoxyadenosinderivat, das durch die Formel

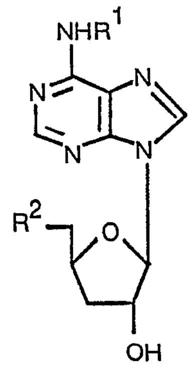

(I)

wiedergegeben wird, in welcher $R^1$ eine Acylgruppe, enthaltend 2 bis 26 Kohlenstoffatome, bedeutet und $R^2$ eine Hydroxylgruppe, einen Phosphorsäurerest oder den Rest eines Phosphorsäuresalzes darstellt, und einen pharmakologisch annehmbaren Träger enthält.

2. Eine Zubereitung wie in Anspruch 1 beansprucht, in welcher der Substituent $R^1$ 2 bis 22 Kohlenstoffatome enthält.

3. Eine chemotherapeutische Zusammensetzung zur Behandlung von Tumoren, welche einen Antitumorwirkstoff, ein Verstärkungsmittel für die Antitumorwirkung, das ein $N^6$-Acyl-3'-deoxyadenosinderivat ist, welches wiedergegeben wird durch die Formel:

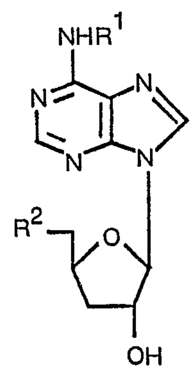

(I)

in welcher $R^1$ eine Acylgruppe, enthaltend 2 bis 26 Kohlenstoffatome, bedeutet und $R^2$ eine Hydroxylgruppe, einen Phosphorsäurerest oder einen Rest eines Phosphorsäuresalzes darstellt, und einen pharmakologisch annehmbaren Träger enthält.

4. Die chemotherapeutische Zusammensetzung zur Behandlung von Tumoren, wie in Anspruch 3 beansprucht, in welcher der Antitumorwirkstoff ausgewählt ist aus Anti-Metaboliten, Antitumor-Antibiotika und Alkylierungsmitteln.

5. Ein $N^6$-Acyl-3'-deoxyadenosinderivat mit der nachstehenden Formel:

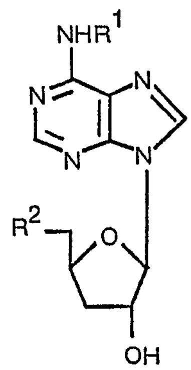

(I)

in welcher $R^1$ eine Acylgruppe, enthaltend 2 bis 26 Kohlenstoffatome, bedeutet und $R^2$ eine Hydroxylgruppe, einen Phosphorsäurerest oder einen Rest eines Phosphorsäuresalzes darstellt, zur Verwendung als ein Verstärkungsmittel für die Antitumorwirkung in einem von einem Tumor befallenden tierischen Lebewesen, welches sich in einer Antitumorbehandlung befindet.

6. Eine Verbindung wie in Anspruch 5 beansprucht zur Verwendung als Verstärkungsmittel in einer

Antitumorbehandlung, welche das Bestrahleneiner von einem Tumor befallenen Stelle des tiereischen Lebewesens umfaßt, wobei das Verstärkungsmittel vor, gleichzeitig mit oder nach der Bestrahlung angewendet wird.

7. Eine Verbindung wie in Anspruch 5 beansprucht zur Verwendung als Verstärkungsmittel in einer Antitumorbehandlung, bevor das tierische Lebewesen der Bestrahlung unterworfen wird.

8. Eine Verbindung wie in Anspruch 5 beansprucht zur Verwendung als ein Verstärkungsmittel in einer Antitumorbehandlung, nachdem das tierische Lebewesen der Bestrahlung unterworfen worden ist.

9. Eine Verbindung wie in Anspruch 5 beansprucht zur Verwendung als ein Verstärkungsmittel in einer Antitumorbehandlung, welche die Verabreichung eines Antitumorwirkstoffes an das tierische Lebewesen umfaßt, wobei das Verstärkungsmittel vor, gleichzeitig mit oder nach der Verabreichung des Antitumorwirkstoffes zur Anwendung kommt.

10. Eine Verbindung wie in Anspruch 5 beansprucht zur Verwendung als Verstärkungsmittel in einer Antitumorbehandlung, in welcher der Antitumorwirkstoff ausgewählt ist aus Anti-Metaboliten, Anti-Tumor-Antibiotika und Alkylierungsmitteln.